# EUROPEAN PATENT SPECIFICATION

(11) **EP 0 423 869 B1**
(45) Date of publication and mention of the grant of the patent: **12.07.1995**
(21) Application number: 90202667.3
(22) Date of filing: 08.10.1990
(51) Int. Cl.: C12N 15/50, C12N 15/86, C12N 15/62, C12P 21/00, A61K 39/215, C07K 14/00

(54) **Infectious bronchitis virus vaccine**
Impfstoff gegen IBV
Vaccin contre le virus de la bronchite infectieuse (IBV)

(30) Priority: 20.10.1989 US 424793
(43) Date of publication of application: 24.04.1991
(73) Proprietor: Akzo Nobel N.V., 6824 BM Arnhem (NL)
(72) Inventor: Sondermeijer, Paulus Jacobus Antonius, NL-5831 RN Boxmeer (NL); Claessens, Johannes Antonius Joseph, NL-5831 MR Boxmeer (NL)
(74) Representative: Hermans, Franciscus G.M.

(56) References cited:
- EP-A- 0 221 609
- EP-A- 0 334 530
- WO-A-86/05806
- WO-A-90/02191
- CHEMICAL ABSTRACTS, vol. 112, 1990, page 274, abstract no. 18635b, Columbus, Ohio, US; L.W. SNEED et al. & VIRAL IMMUNOL. 1989, 2(3), 221-7

## Description

The present invention is concerned with a nucleic acid sequence encoding a spike protein polypeptide of an Infectious Bronchitis Virus (IBV), a recombinant nucleic acid molecule comprising such a nucleic acid sequence, a vector or a host cell containing said nucleic acid sequence, a polypeptide comprising an amino acid sequence of a spike protein polypeptide of IBV, an antibody or antiserum immuno-reactive with said polypeptide as well as a vaccine for the protection of animals against IBV infection.

The IB virus causes an acute, highly contagious disease of chickens, characterized by typical respiratory symptoms such as tracheal rales, gasping, coughing and nasal discharge. IB can cause high mortality, particular in young chickens. Moreover, kidneys and reproductive tract may be affected, the latter damage can result in a drop in egg production in layer or breeder hens. IB can predispose chickens, especially broilers to infection by certain strains of E. coli resulting in an increased mortality.

The chicken was considered to be the only host of the IB virus, but recently the virus has also been isolated from turkeys.

IBV is a member of the genus Coronaviridae, a group of enveloped viruses containing a genome consisting of a single-stranded RNA of about 20 kb. This genome encodes inter alia three important structural proteins: a spike protein (S), a membrane protein (M) and a nucleocapsid protein (N). The 155 kD precursor for the glycosylated spike protein is cleaved after translation in two structurally unrelated subunits S₁ and S₂. Two or three copies of each of S₁ and S₂ form a characteristic IBV surface structure, the spike or peplomer. The spike protein and the subunit fragments thereof play an important role in inducing circulating virus neutralizing antibodies in infected birds.

At present chickens can be protected against IBV infection by live attenuated virus vaccines. However, this type of vaccine suffers from a number of drawbacks including low stability and possible adverse effects on the kidneys and respiratory and reproductive tracts. Moreover, using attenuated live vaccines always involve the risk of inoculating animals with partially attenuated pathogenic viruses. In addition the attenuated viruses may revert to a virulent state resulting in disease of the inoculated animals and the possible spread of the virulent virus in the flock.

Another problem encountered with the use of live virus vaccine is the possible contamination by other viruses in cell cultures used to grow the vaccine virus.

Inactivated virus vaccines generally induce only a low level of immunity. Especially if a local protective activity in the respiratory or intestinal tract is desired, as in the present case, such a vaccine requires additional immunizations (boosters). Furthermore, the neutralisation-inducing antigenic determinants of the virus may become altered by the inactivation treatment, decreasing the protective potency of the virus.

Vaccine viruses presently used for the combatment of IB have been selected both for their immunogenic properties on the base of their antigenic spectrum and reduced pathogenicity (live virus vaccine). Vaccines may contain either a particular serotype such as Connecticut (e.g. Connecticut isolate A 5968) or Massachusetts (e.g. strains Beaudette, M41 and M42) protecting only against the homologous type or may use a particular virus strain shown to have a broader antigenic spectrum such as the Holland (H) strains, e.g. H120 and Ma5. However, it is known that aforementioned vaccines do not provide substantial immunity against infection with IB viruses belonging to the Arkansas serotype, requiring the additional vaccination of birds with an Arkansas serotype IBV strain. The existence of this serotype is for the first time described by D.B. Fields (1973).

According to the present invention a nucleic acid sequence substantially encoding the S₁ subunit of the spike protein polypeptide of an IBV strain belonging to the Arkansas serotype can be applied for the preparation of a vaccine for the immunization of poultry against infection of birds with an Arkansas serotype IBV strain and which does not display above-mentioned drawbacks of live attenuated or inactivated IBV vaccines.

In order to determine whether a specific virus strain belongs to the Arkansas serotype, i.e. belongs to the same serotype as the IBV strain DPI 3168, the virus-neutralization test described in Cowen and Hitchner (1975) and in Gelb et al. (1981) should be used.

"Nucleic acid sequence" as used herein refers to a polymeric form of nucleotides of any length, both to ribonucleic acid sequences and to deoxyribonucleic acid sequences. In principle, this term refers to the primary structure of the molecule. Thus, this term includes double- and single stranded DNA, as well as double- and single stranded RNA and modifications thereof.

Particularly, a nucleic acid sequence according to the present invention can be used that substantially encodes a spike protein polypeptide of strain DPI 3168 from IBV.

A preferred nucleic acid sequence to be used according to the invention substantially encodes a polypeptide with an amino acid sequence 1-1168, shown in SEQ ID No:1.

A nucleic acid sequence according to the invention encoding subunit S₁ of a spike protein polypeptide of an IBV strain belonging to the Arkansas serotype, forms part of the invention.

Particularly a nucleic acid sequence according to the invention substantially encoding the subunit of a spike protein polypeptide, said subunit having an amino acid coding region corresponding with amino acid sequence 1-539 shown in SEQ ID No:1, is included within the scope of the invention.

The joining region, i.e. a nucleic acid sequence encoding an amino acid sequence linking the S₁ and S₂ subunits, in particular the amino acid sequence 540-544 shown in SEQ ID No:1 may also form part of the nucleic acid sequence substantially encoding the S₁ subunit or the amino acid sequence about 1-539, respectively. Such a nucleic acid sequence encodes antigenic fragments of this subunit, which can be used for the preparation of a vaccine for the immunization of poultry against IBV infection or diagnostic purposes.

Various methods are known for detecting such usable polypeptide fragments (termed epitopes) within a known amino acid sequence. On the basis of a known amino acid sequence, these epitopes can, for example, be determined experimentally with the aid of the screening techniques described in patent publications WO 84/03564 and WO 86/06487.

In addition, a number of regions of the polypeptide, with the stated amino acid sequence, can be designated epitopes on the basis of theoretical considerations and structural agreement with epitopes which are now known. The determination of these regions can be based on a combination of the hydrophilicity criteria according to J.P. Hopp and K.R. Woods (1981) and the secondary structure aspects according to P.Y. Chou and G.D. Fasman (1987).

Another method to locate an epitope containing region is the use of so called "mar" mutants (monoclonal antibody resistant mutants). Nucleic acid sequence analysis of specific parts of the genome of variant IBV viruses which resist neutralizing monoclonal antibodies can reveal the position within a polypeptide which is essential for the neutralization-inducing activity of said polypeptide.

The following regions contain epitopes which are important for the neutralizing activity of the antibody response against a spike protein polypeptide, inter alia:
Val₅₂ - Tyr₇₂
Gly₁₁₈ - His₁₄₇.

A deoxynucleic acid sequence encoding a spike protein polypeptide of IBV strain DPI 3168 is shown in SEQ ID No:1. This cDNA sequence is 3504 nucleotides in length. The sequence fragments between nucleic acid number 151-1767 and 1783-3654 encode the S₁ and S₂ subunits respectively, said sequences being linked by a small joining region.

A preferred nucleic acid sequence according to the invention comprising genetic information encoding a fragment of the spike protein polypeptide substantially encodes the S₁ subunit and more in particular comprises the nucleic acid sequence 151-1767 shown in SEQ ID No:1.

The information provided herein, although derived from one strain of an IBV belonging to the Arkansas serotype, is sufficient to allow a person skilled in the art to isolate and identify nucleic acid sequences encoding a spike protein polypeptide derived of other naturally occurring variant IBV strains belonging to the Arkansas serotype, e.g. Arkansas 99, or an antigenic fragment thereof.

The cDNA sequence shown in SEQ ID No:1, or a fragment thereof can be used for this purpose. Such a nucleic acid sequence can be used to screen cDNA libraries, by hybridization under conditions of appropriate stringency, prepared from the genomic RNA of other IBV strains belonging to the Arkansas serotype (Slater, R.J., 1986, chap. 5 and 8; Singer-Sam, J. et al., 1983; Maniatis, T. et al., 1989).

Both methods for constructing cDNA libraries and hybridization techniques are outlined herein.

As is well known in the art, the degeneracy of the genetic code permits substitution of bases in a codon resulting in an other codon but still coding for the same amino acid, e.g. the codon for the amino acid glutamic acid is both GAT and GAA. Consequently, it is clear that for the expression of a polypeptide with the amino acid sequence shown in SEQ ID No:1, or an antigenic fragment thereof use can be made of a nucleic acid sequence with such an alternative codon composition different from the nucleic acid sequence shown in SEQ ID No:1.

Also included within the scope of the invention is a nucleic acid sequence which hybridizes under stringent conditions with a nucleic acid sequence shown in SEQ ID No:1. Said hybridizable nucleic acid sequence displays a substantial homology with a nucleic acid sequence shown in SEQ ID No:1, or with a fragment thereof but may comprise nucleotide substitutions, mutations, insertions, deletions, inversions etc. and encodes a protein or polypeptide which is functionally equivalent to a spike protein polypeptide of an IBV belonging to the Arkansas serotype, or an antigenic fragment thereof, i.e. the amino acid sequence of such a related polypeptide is not identical with the amino acid sequence of a spike protein polypeptide of IBV strain DPI 3168, or an antigenic fragment thereof, but features corresponding immunological properties characteristic for a spike protein polypeptide of an IBV strain belonging to the Arkansas serotype or an antigenic fragment thereof.

It will be understood that for the particular polypeptide of IBV strain DPI 3168 embraced herein, natural variations can exist between individual DPI 3168 viruses or strains of the Arkansas serotype. These variations may be demonstrated by (an) amino acid difference(s) in the overall sequence or by deletions, substitutions, insertions, inversions or additions of (an) amino acid(s) in said sequence. Nucleic acid sequences encoding such derivatives are included within the scope of this invention. Moreover, the potential exist to use recombinant DNA technology for the preparation of nucleic acid sequences encoding these various derivatives.

Preferably, nucleic acid sequences according to the invention may be derived from available isolates of the DPI 3168 strain.

All such modifications resulting in derivatives of the nucleic acid sequence shown in SEQ ID No:1 are included within the ambit of this invention.

The present invention comprises also a polypeptide of an IBV strain which is encoded by a nucleic acid sequence mentioned above and which can be used for the immunization of poultry against IB.

Furthermore, a polypeptide substantially comprising the amino acid sequence of a spike protein polypeptide of an IBV belonging to the Arkansas serotype, especially of the DPI 3168 strain, preferably the S₁ subunit is included in the present invention.

In a preferred embodiment a polypeptide substantially comprising the amino acid sequence about 1-1168, shown in SEQ ID No:1, or a fragment thereof, e.g. amino acid sequence about 1-539, is used.

In particular a polypeptide comprising amino acid sequence about 1-539, shown in SEQ ID No:1, is used in the present invention.

The term polypeptide refers to a molecular chain of amino acids and does not refer to a specific length of the product; thus, inter alia peptides oligopeptides and proteins are included within the definition of polypeptide.

It will be understood that for the particular spike protein polypeptide shown in SEQ ID No:1, embraced herein, natural variations can exist. These variations may be demonstrated by (an) amino acid difference(s) in the overall sequence or by deletions, substitutions, insertions, inversions or additions of (an) amino acid(s) in said polypeptide.

Moreover, the potential exists in the use of recombinant DNA technology for the preparation of various derivatives of the spike protein polypeptide shown in SEQ ID No:1, variously modified by resultant single or multiple amino acid substitutions, deletions, additions or replacements. All abovementioned modifications resulting in derivatives of the spike protein polypeptide shown in SEQ ID No:1 are included within the scope of this invention so long as the essential, characteristic activity of the polypeptide shown in SEQ ID No:1 or an antigenic fragment thereof, remains unaffected in essence.

A nucleic acid sequence according to the present invention can be ligated to various replication effecting DNA sequences, optionally containing portions of DNA encoding fusion protein sequences such as β-galactosidase, resulting in a so called recombinant nucleic acid molecule which can be used for the transformation of a suitable host. Such hybrid DNA molecules, are preferably derived from, for example plasmids, or from nucleic acid sequences present in bacteriophages, cosmids or viruses. Specific vectors which can be used to clone nucleic acid sequences according to the invention are known in the art (e.g. Rodriquez, R.L. and D.T. Denhardt, 1988). The methods to be used for the construction of a recombinant nucleic acid molecule according to the invention are known to those of ordinary skill in the art and are inter alia forth in Maniatis, T. et al. (1982). "Transformation", as used herein, refers to the introduction of a heterologous nucleic acid sequence into a host cell, irrespective of the method used, for example direct uptake or transduction. The heterologous nucleic acid sequence may be maintained through autonomous replication or alternatively, may be integrated into the host genome. If desired, the recombinant DNA molecules are provided with appropriate control sequences compatible with the designated host which can regulate the expression of the inserted nucleic acid sequence.

A suitable host cell is a cell which can be transformed by a nucleic acid sequence encoding a polypeptide or by a recombinant nucleic acid molecule comprising such a nucleic acid sequence and which can if desired be used to express said polypeptide encoded by said nucleic acid sequence. The host cell can be of procaryotic origin, e.g. bacteria such as E.coli, B.subtilis and Pseudomonas species; or of eucaryotic origin such as yeasts, e.g. Saccharomyces cerevisiae or higher eucaryotic cells such as insect, plant or mammalian cells, including HeLa cells and Chinese hamster ovary (CHO) cells. Insect cells include the Sf9 cell line of Spodoptera frugiperda. Information with respect to the cloning and expression of the nucleic acid sequence of the present invention in eucaryotic cloning systems can be found in Esser, K. et al. (1986).

In general, prokaryotes are preferred for cloning of DNA sequences in constructing the vectors useful in the invention.

For expression, nucleic acid sequences of the present invention are operably linked to expression control sequences. Such control sequences may comprise promoters, operators, enhancers, inducers, ribosome binding sites etc.

When the host cells are bacteria, illustrative useful expression control sequences include the trp promoter and operator (Goeddel, et al., 1980); the lac promoter and operator (Chang et al., 1978); the outer membrane protein promoter (Nakamura and Inouge, 1982); the bacteriophage λ promoters and operators (Remaut, E. et al., 1983); the α-amylase (B.subtilis) promoter and operator, termination sequence and other expression enhancement and control sequences compatible with the selected host cell. When the host cell is yeast, illustrative useful expression control sequences include, e.g., α-mating factor. For insect cells the polyhedrin promoter of baculoviruses can be used (Smith, G.E. et al., 1983). When the host cell is of mammalian origin illustrative useful expression control sequences include, e.g., the SV-40 promoter (Berman, P.W. et al., 1983) or e.g. the metallothionein promoter (Brinster, R.L. et al., 1982) or a heat shock promoter (Voellmy et al., 1985). Alternatively, also expression control sequence present in IBV, in particular those regulating the expression of the DPI 3168 spike protein may be applied.

Immunization of poultry against IBV infection can, for example be achieved by administering to the bird a polypeptide according to the invention as a so-called subunit vaccine. The subunit vaccine according to the invention may comprise a polypeptide in a pure form, optionally in the presence of a pharmaceutically acceptable carrier. The polypeptide can optionally be covalently bonded to a non-related protein, which, for example can be of advantage in the purification of the fusion product. Examples are β-galactosidase, protein A, prochymosine, blood clotting factor Xa, etc.

In some cases the ability to raise neutralizing antibodies against these polypeptides per se may be low. Small fragments are preferably conjugated to carrier molecules in order to raise their immunogenicity. Suitable carriers for this purpose are macromolecules, such as natural polymers (proteins, like key hole limpet hemocyanin, albumin, toxins), synthetic polymers like polyamino acids (polylysine, polyalanine), or micelles of amphiphilic compounds like saponins. Alternatively these fragments may be provided as polymers thereof, preferably linear polymers.

Polypeptides to be used in such subunit vaccines can be prepared by methods known in the art, e.g. by isolation said polypeptides from IBV, by recombinant DNA techniques or by chemical synthesis.

If required the polypeptides according to the invention to be used in a vaccine can be modified in vitro or in vivo, for example by glycosylation, amidation, carboxylation or phosphorylation.

An alternative to subunit vaccines are live vector vaccines. A nucleic acid sequence according to the invention is introduced by recombinant DNA techniques into a micro-organism (e.g. a bacterium or virus) in such a way that the recombinant micro-organism is still able to replicate thereby expressing a polypeptide coded by the inserted nucleic acid sequence. Next, this recombinant micro-organism can be administered to the bird for immunization whereafter it maintains itself for some time, or even replicates, in the body of the inoculated bird, expressing in vivo a polypeptide coded for by the inserted nucleic acid sequence according to the invention resulting in the stimulation of the immune system of the inoculated bird. Suitable vectors for the incorporation of a nucleic acid sequence according to the invention are derived from, for example pox viruses such as avian pox virus, e.g. fowl pox virus, herpes viruses such as Marek's disease virus or herpes virus of turkey, adeno virus, influenza virus, or bacteria such as E. coli or specific Salmonella species. With recombinant micro-organisms of this type, the polypeptide synthesized in the host cell can be exposed as a surface antigen. In this context fusion of the said polypeptide with OMP proteins or pilus proteins of Escherichia coli or synthetic provision of signal and anchor sequences which are recognized by the organism are conceivable. It is also possible that the said immunogenic polypeptide, if desired as part of a larger whole, is released inside the animal to be immunized. In all of these cases it is also possible that one or more immunogenic products will find expression which generate protection against various pathogens and/or against various antigens of a given pathogen.

A vaccine according to the invention can be prepared by culturing a host cell infected with the vector virus comprising a nucleic acid sequence according to the invention, whereafter vector viruses grown in the cells can be collected, optionally in the presence of the cells or in a pure form, and formed to a vaccine optionally in a lyophilized form.

Abovementioned host cells comprising a nucleic acid sequence according to the invention can also be cultured under conditions which are favourable for the expression of a polypeptide coded by said nucleic acid sequence. Vaccines may be prepared using samples of the crude culture, host cell lysates or host cell extracts, although in another embodiment more purified polypeptides according to the invention are formed to a vaccine, depending on its intended use. In order to purify the polypeptides produced, host cells containing a nucleic acid sequence according to the invention are cultured in an adequate volume and the polypeptides produced are isolated from such cells or from the medium if the protein is excreted. Polypeptides excreted into the medium can be isolated and purified by standard techniques, e.g. salt fractionation, chromatography, centrifugation, whereas intracellular polypeptides can be isolated by first collecting said cells, lysing the cells followed by separation of the polypeptides from the other intracellular components and forming the polypeptides to a vaccine.

It goes without saying that birds already infected by IBV can be treated with antibodies directed against said IBV. Antiserum or antibodies characteristic for a polypeptide according to the invention can be used for the therapeutic treatment of IBV infection. Said characteristic antiserum or antibodies may be obtained by incubating antiserum evoked against a polypeptide of the present invention with a mixture of IB viruses of other known serotypes. Antibodies not characteristic for a polypeptide according to the invention adsorb to the added virus material and can thus be separated, e.g. by centrifugation, from the incubation mixture resulting in a polyclonal antibody preparation characteristic for a polypeptide according to the invention.

Monoclonal antibodies directed against a polypeptide according to the invention can also be used for the therapy of birds infected with IBV. Said monoclonal antibodies can be produced by methods known in the art for this purpose, e.g. by immunizing mice with said polypeptide, immortalizing mouse spleen cells and selecting hybridomas producing useful antibodies. Immortal antibody-producing cell lines can also be created by direct transformation of B lymphocytes with oncogenic DNA, or transfection with Epstein-Barr virus.

Monoclonal antibodies, in particular, may be used to raise anti-idiotype antibodies by methods known in the art. These anti-idiotype antibodies may also be useful for prevention of IBV infection in birds.

Abovementioned antiserum and monoclonal antibodies can also be used for the immunological diagnosis of birds infected with IBV.

The vaccine according to the invention can be administered in a conventional active immunization scheme: single or repeated administration in a manner compatible with the dosage formulation and in such amount as will be prophylactically and/or therapeutically effective and immunogenic. The administration of the vaccine can be done, e.g. intradermally, subcutaneously, intramusculary, intra-venously or intranasally.

Additionally the vaccine may also contain an aqueous medium or a water containing suspension, often mixed with other constituents, e.g. in order to increase the activity and/or shelf life. These constituents may be salts, pH buffers, stabilizers (such as skimmed milk or casein hydrolysate), emulsifiers, adjuvants to improve the immune response (e.g. mineral oils, muramyl dipeptide, aluminium hydroxide, saponin, polyanions and amphipatic substances) and preservatives.

It is clear that a vaccine according to the invention may also contain immunogens related to other IBV serotypes or to other diseases or may contain nucleic acid sequences encoding these immunogens, like antigens of IBV of the Massachusetts serotype, Newcastle disease virus (NDV), Infectious Bursal Disease Virus (IBDV) and Marek's Disease Virus (MDV), to produce a multivalent vaccine.

### Example 1

### Preparation of genomic viral RNA

Virus from the 19th passage of IBV strain DPI 3168 (Gelb et al., 1981 and Gelb and Cloud, 1983) was grown in 10 day old embryonated eggs, by inoculating the allantoic cavity with 10⁴ EID₅₀/egg. After 24 h. incubation at 37 ^{o}C egg's were chilled overnight at 4 ^{o}C. Allantoic fluid was harvested taking care to keep it cool on ice. Red blood cells and debris were removed by centrifugation at 4 ^{o}C and 6000 x g. for 30′.

Virus was pelleted from the supernatant at 54.000 x g in a Beckmann Type 19 rotor for 4 h. at 4 ^{o}C. Pellet was resuspended in cold TNE (10 mM Tris-HCl, 100 mM NaCl, 1 mM EDTA, pH 7.5) by repeated passage through a syringe needle and layered onto a 32 ml lineair gradient of 20-60% sucrose in TNE.

After overnight centrifugation at 4 ^{o}C in a SW28 rotor at 24.000 rpm, virus band was collected through the side wall puncturing the tube with a syringe. After dilution with 2 volumes TNE, virus was pelleted in a SW 28 rotor at 18.000 rpm for 90′ at 4 ^{o}C.

Material was resuspended in a small volume of TNE and sodium dodecylsulphate was added to a final concentration of 0.5%. Preparation was digested with proteinase K (Boehringer) at 200 »g/ml for 2 h. at 37 ^{o}C and extracted twice with a 1:1 mixture of phenol/chloroform.

Viral RNA in the aqueous phase was precipitated with 2 volumes of ethanol in the presence of 0.1 M sodium acetate pH 6.0 at -20 ^{o}C. After centrifugation and rinsing the tube with ethanol, pellet was dried under vacuum and dissolved in sterile water to give an RNA concentration of 0.5 mg/ml. Preparation contained >90% of IBV genomic RNA as checked by agarose gel electrophoresis and was stored at -20 ^{o}C.

### cDNA cloning of genomic RNA

First strand synthesis was primed with oligo (dT)₁₂₋₁₈ in the presence of AMV reverse transcriptase using 5 »g of viral RNA in a 75 »l reaction volume. After incubating 30′ at 44 ^{o}C, DNA/RNA hybrids were denatured by heating 3′ at 100 ^{o}C followed by synthesis of the second strand in the presence of the large fragment from E. coli DNA polymerase I incubating the reaction for 2 h. at 20 ^{o}C. cDNA was precipitated with ethanol and digested with 10 U. of S₁-nuclease in a 200 »l reaction volume for 30′ at 37 ^{o}C. Reaction products were layered onto 3.2 ml of a 5-20% sucrose gradient in 10 mM Tris-HCl, 5 mM EDTA, 500 mM NaCl, pH 7.5 and centrifuged in a SW65 rotor at 30.000 rpm for 16 h. at 15 ^{o}C.

Material sedimenting with a size between 500 and 5000 basepairs was collected, ethanol precipitated and dissolved in 20 »l of 0.1 SSC (15 mM NaCl, 1.5 mM sodium citrate).

Ends of the double stranded cDNA were extended with 10 to 15 dG residues by a 2′ incubation at 37 ^{o}C with 15 U. terminal transferase (Gibco-BRL) in a 30 »l reaction volume according to the conditions recommended by the enzyme supplier. Reaction was stopped with 5 mM EDTA.

Ten nanograms of tailed cDNA were heated for 2′ at 65 ^{o}C with a 25-molar excess of the phosphorylated synthetic oligomer 5′-dAATTCCCCCCCCCCC-3′ in a final volume of 10 »l TEN and annealed together by overnight incubation at 50 ^{o}C.

Ligation with 10 »g of EcoRI digested λgt10 DNA (Huynh et al., 1985) was in 20 »l of 30 mM Tris-HCl, pH 7.5, 10 mM MgCl₂, 10 mM DTT, 0.1 mM ATP, adding 1 U. of T₄ DNA ligase and incubating overnight at 4 ^{o}C. DNA was added to in vitro packaging reaction mixture (Promega) and a cDNA library from IBV strain DPI 3168 was established by selecting for recombinant phages after plating on a hfl A strain of E. coli.

### Isolation of cDNA clones encoding fragments of the spike protein

One to two hundred pfu. of the cDNA library were plated in a petridish on a lawn of E. coli. Duplicate filters of nitrocellulose were prepared (Benton and Davis, 1978) and incubated overnight at 42 ^{o}C with ³²P-labeled synthetic oligomers in a hybridization solution containing 10 mM Tris-HCl, pH 7.5, 1 M NaCl, 0.1% SDS and 4 x Denhardt's solution (Maniatis et al., 1982).

The three synthetic oligomers used as probes in these hybridizations contained the following nucleotide sequence structure:
I. 5′-dTTCCAACATCTCTAACCAGTAATTTACCGT-3′
II. 5′-dTACCTACTAATTTACCACCAGAAACTACAAACTGCTG-3′
III. 5′-dTGGATCATTAAACAGACTTTTTAGGTCTGTATTGTT-3′

Recombinant phages giving a signal with one or preferentially two of these probes were selected and plaque purified by standard procedures (Maniatis et al., 1982). cDNA fragments from λ phage recombinants were flanked by EcoRI restriction sites and transferred as such into the EcoRI site from plasmid cloning vector pGEM4Z (Promega).

Restriction analysis and partial sequencing on two candidates showed that one encoded the complete S₁ and the other encoded the S₂ moiety of the spike gene.

The sequence of these two DNA fragments partially overlapped with each other in particular with respect to the unique XbaI-restriction site at the S₁/S₂ junction. This site was then used to assemble the two fragments mentioned above and resulted in plasmid construction pIB14 carrying the complete gene encoding the spike protein polypeptide from IBV strain DPI 3168 in plasmid vector pGEM4Z.

### DNA-sequencing

In these experiments the enzyme Exonuclease-III was used to remove increasing parts of one end of the S-gene according to Henikoff (1984). To prepare the S-gene from DPI 3168 for this technique, the EcoRI-HindIII fragment of pIB14 was inserted into EcoRI-HindIII digested and defosforylated pGEM7Zf+ vector plasmid. This construct was denominated pIB15. 5 »g of pIB15 was digested with ApaI, leaving 3′ overhanging ends preventing Exo-III from digesting the T7-polymerase primer site, and EcoRI, generating 5′ overhanging ends which are available for the Exo-III digestion. At 30 second intervals, samples were taken from the digestion mixture. The samples were treated with S₁-nuclease and Klenow enzyme to yield blunt-ended DNA fragments. The fragments were circularised in the presence of T₄-DNA ligase and transformed into competent E. coli cells.

After screening of the ampicilline resistant E. coli colonies, 120 S-gene fragments were selected that covered almost the entire spike gene. DNA-sequencing was performed as published by Sanger et al. (1977), using the dideoxy chain-termination technique directly on double stranded plasmid DNA from minipreparations. Sequence was primed both from the T7 and Sp6 promoter sites in pGEM7Zf+ and reaction products were visualized by autoradiography using [α³²-P]dATP in the labelling reaction.

The gap that remained in the sequence between nucleotides 2500 and 2600 was filled using a 15 base synthetic primer which hybridised at pos. 2834. The sequence data were collected, assembled and analysed on an IBM PC using the Gene-Master programs (Bio-Rad).

### Example 2

### Insertion of the gene encoding the spike protein of IBV strain DPI 3168 into the viral genome of herpes virus of turkey (HVT).

Based on the genome structure of HVT as published by Igarashi et al. (1987), a region in the unique-short sequence element (Us) of the virus is selected for the insertion of foreign genes. The corresponding DNA fragment is screened from a λEMBL3 library constructed by partially digesting total DNA from HVT infected CEF.

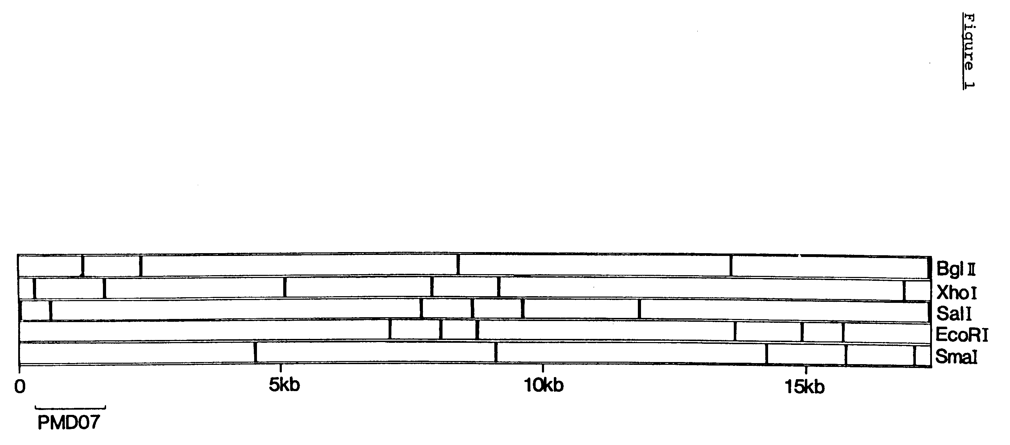

The insert of one of the λ-isolates, characterized by the absence of any BamHI restriction site, is denominated λHVT04 and analyzed in detail by physical mapping (Figure 1). The sequence present in the 17.5 kb inserted fragment represents a major part of the Us region including part of the inserted repeat structure (Igarashi et al., 1987).

One of the 1.2 kb Xho I restriction fragments from λHVT04 is subcloned in pGEM3Z digested with Sal I resulting in plasmid pMDO7 which contains a unique BglII site available for insertion of DNA fragments.

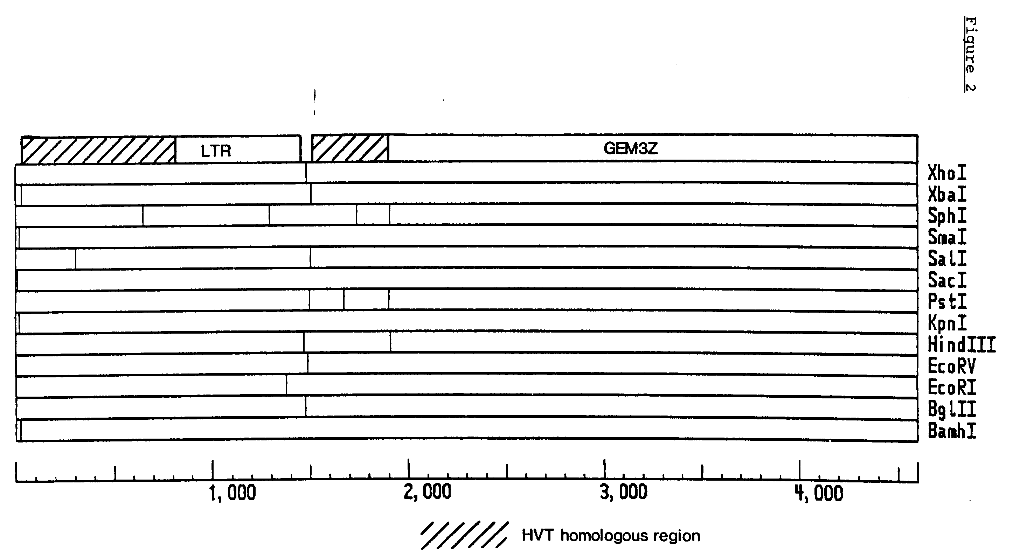

A strong promoter which could direct the expression of foreign genes after their insertion into the genome of the HVT virus is selected from the long terminal repeat (LTR) sequence of Rous sarcoma virus (RSV). The promoter has been mapped on a 580 bp NdeI/HindIII restriction fragment from pRSVcat (Gorman et al., 1982) and can be inserted between the HindIII and PstI sites of pGEM3Z (Promega, Madison, USA) by means of double stranded synthetic linkers on both sides of the fragment. The connection between the HindIII site from the vector pGEM3Z and the NdeI site of the RSV fragment carrying the LTR-promoter is made with a 30 bp linker containing cohesive ends compatible with HindIII on one and NdeI on the other site. However, after ligation both restriction sites are not restored due to deliberate modifications in the outer nucleotides of the six basepair recognition sequence. In addition to the removal of these two sites, a new restriction site (BamHI) present within the linker itself is created at the corresponding position. A second 20 bp linker is synthesized which connects the HindIII site from the LTR fragment to the PstI site from pGEM3Z, in this case without destruction of the recognition sequence on either of the ends and adding the three convenient unique restriction sites BglII, XhoI and EcoRV, to those already present in the polylinker of pGEM3Z, e.g. PstI, SalI, XhoI and BamHI. The resulting derivative of pGEM3Z, designated pVECO1, therefore contains a 650 bp restriction fragment carrying the LTR promoter sequence immediately followed by seven restriction sites available for the insertion of foreign genes. The 650 bp fragment is flanked on either end by a BamHI restriction site and is transferred as such to the unique BglII site present in the 1.2 kb HVT insert from pMDO7. The cohesive ends generated by these two restriction enzymes are compatible but ligation does not restore either of the original recognition sequences for BglII or BamHI. One of the resulting constructs, carrying the LTR in the orientation towards the TRₛ, is designated pVECO4, and checked by restriction mapping (Figure 2). The structure of this universal HVT recombination vector allows the insertion of foreign genes immediately downstream of the LTR promoter and subsequent integration of the complete expression cassette into the HVT genome by in vivo recombination. The positions of the different restriction sites downstream of the LTR in particular those for the enzymes BglII, XhoI and EcoRV are designed in such a way that even multiple gene insertion can be envisaged. A 3.8 kb SalI/XhoI restriction fragment from pIB15 carrying the spike gene from DPI 3168, was inserted into the unique XhoI site of pVECO4 downstream of the LTR promoter. One of the candidates having the gene inserted in the correct orientation relative to the LTR promoter, is analyzed by restriction mapping in order to confirm correct structure. This plasmid is designated pIB27 and used subsequently in the co-transfection of chicken embryo fibroblasts (CEF).

Recombinant HVT virus is identified by immunofluorescence staining of infected cell cultures with an antibody probe which specifically recognizes the spike protein from IBV strain DPI 3168. Establishment of a homogeneous recombinant HVT virus preparation is done by standard procedures, such as limiting dilution techniques or single plaque isolation.

### Legends

### Figure 1

Restriction enzyme map of 17.5 kb insert of λHVTO4, indicating also the BglII site containing XhoI fragment present in pMDO7.

### Figure 2

Restriction enzyme map of pVECO4 showing the LTR-promoter inserted into the unique BglII site of the 1.2 kb XhoI HVT fragment from pMDO7.

### References

1. Benton, W.D., and Davis, R.W. (1978), Science 196, 180.
2. Berman, P.W. et al. (1983), Science 222, 524-527.
3. Brinster, R.L. et al. (1982), Nature 296, 39-42.
4. Chang et al. (1978), Nature 275, 615.
5. Chou, P.Y., et al. (1987), Advances in Enzymology 47, 45.
6. Cowen, B.S. and Hitchner, S.B., (1975), Avian Diseases 19, 583.
7. Esser, K. et al. (1986), Plasmids of Eukaryotes, Springer-Verlag.
8. Fields, D.B. (1973), Avian Diseases 17, 659.
9. Gelb, J., et al. (1981), Avian Diseases 25, 655.
10. Gelb, J. and Cloud, S.S. (1983), Avian Diseases 27, 679.
11. Goeddel et al. (1980), Nucl. Acids Res. 8, 4057.
12. Gorman, L. et al. (1982), Proc.Natl.Acad.Sci. U.S.A. 79, 6777.
13. Henikoff, S. (1984), Gene 28, 351.
14. Hopp, J.P., et al. (1981), Proc.Nat.Acad.Sci. U.S.A. 78, 3824.
15. Huynh, T.V., et al. (1985), in "DNA cloning, Vol. I", ed. D. Glover, 49.
16. Igarashi, T. et al. (1987), Virology 157, 351.
17. Maniatis, T., et al. (1982), in "Molecular cloning", Cold Spring Harbor Laboratory Press, U.S.A.
18. Maniatis, T., et al. (1989), in "Molecular cloning", Cold Spring Harbor Laboratory Press, U.S.A.
19. Nakamura, K. and Inouge, M. (1982), EMBO J. 1, 771-775.
20. Remaut, E. et al. (1983), Nucl. Acids Res. 11, 4677-4688.
21. Rodriquez, R.L. and Denhardt, P.T. (1988), Vectors: A survey of molecular cloning vectors and their uses, Butterworths.
22. Sanger, I., et al. (1977), Proc.Nat.Acad.Sci U.S.A. 74, 5463.
23. Singer-Sam, J. et al. (1983), Proc.Natl.Acad.Sci. U.S.A. 80, 802-806.
24. Slater, R.J., ed.,(1986), Experiments in Molecular Biology, Clifton, U.S.A.
25. Smith, G.E. et al. (1983), Mol.Cell.Biol. 3, 2156-2165.
26. Voellmy et al. (1985), Proc.Natl.Acad.Sci. U.S.A. 82, 4949-4953.

Sequence Listing

SEQ ID NO : 1
Sequence type : nucleotide with corresponding
protein
Sequence length : 3824 base pairs; 1168 amino acids

Strandness : source is single stranded
Topology : linear
Molecule type : cDNA to genomic RNA

Original source
Organism : Infectious Bronchitis Virus
Immediate experimental
source : λgt10 cDNA library

Properties : spike protein.

AATTCCCCCC CCCAACCTCA GCTTATAGTA TATTTGACGT TGCTAAGTTT GATTTGAAAT 60

TAAAAGCAAC GCCAGTTGTA AATTTGAAAA CTGAACAAAA GACCGACTTA GTAGTTAATT 120

1 5 10
Met Leu Val Lys Ser Leu Phe Leu Val Thr
TACTAAGGAA CGGTAAATTG TTAGTTAGAG ATG TTG GTG AAG TCA CTG TTT CTA GTG ACC 180

15 20 25
Ile Leu Phe Ala Leu Cys Ser Ala Asn Leu Tyr Asp Asn Glu Ser Phe Val Tyr Tyr Tyr
ATT TTG TTT GCA CTA TGT AGT GCT AAT TTA TAT GAC AAC GAA TCT TTT GTG TAT TAC TAC 240

35 40 45
Gln Ser Ala Phe Arg Pro Gly His Gly Trp His Leu His Gly Gly Ala Tyr Ala Val Val
CAG AGT GCT TTT AGG CCA GGA CAT GGT TGG CAT TTA CAT GGA GGT GCT TAT GCA GTA GTT 300

55 60 65
Asn Val Ser Ser Glu Asn Asn Asn Ala Gly Thr Ala Pro Ser Cys Thr Ala Gly Ala Ile
AAT GTG TCT AGT GAA AAT AAT AAT GCA GGT ACT GCC CCA AGT TGC ACT GCT GGT GCT ATT 360

75 80 85
Gly Tyr Ser Lys Asn Leu Ser Ala Ala Ser Val Ala Met Thr Ala Pro Leu Ser Gly Met
GGC TAC AGT AAG AAT CTC AGT GCG GCC TCA GTA GCC ATG ACT GCA CCA CTA AGT GGT ATG 420

95 100 105
Ser Trp Ser Ala Asn Ser Phe Cys Thr Ala His Cys Asn Phe Thr Ser Tyr Ile Val Phe
TCA TGG TCT GCC AAC TCT TTT TGT ACA GCC CAC TGT AAT TTT ACT TCT TAT ATA GTG TTT 480

115 120 125
Val Thr His Cys Tyr Lys Ser Gly Ser Asn Ser Cys Pro Leu Thr Gly Leu Ile Pro Ser
GTT ACA CAT TGT TAT AAG AGC GGA TCT AAT AGT TGT CCT TTG ACA GGT CTT ATT CCA AGC 540

135 140 145
Gly Tyr Ile Arg Ile Ala Ala Met Lys His Gly Ser Ala Met Pro Gly His Leu Phe Tyr
GGT TAT ATT CGT ATT GCT GCT ATG AAA CAT GGA AGT GCT ATG CCT GGT CAC TTA TTT TAT 600

155 160 165
Asn Leu Thr Val Ser Val Thr Lys Tyr Pro Lys Phe Arg Ser Leu Gln Cys Val Asn Asn
AAT TTA ACA GTT TCT GTG ACT AAA TAT CCT AAG TTT AGA TCG CTA CAA TGT GTT AAT AAT 660

175 180 185
His Thr Ser Val Tyr Leu Asn Gly Asp Leu Val Phe Thr Ser Asn Tyr Thr Glu Asp Val
CAT ACT TCT GTA TAT TTA AAT GGT GAC CTT GTT TTC ACA TCT AAC TAT ACT GAA GAT GTT 720

195 200 205
Val Ala Ala Gly Val His Phe Lys Ser Gly Gly Pro Ile Thr Tyr Lys Val Met Arg Glu
GTA GCT GCA GGT GTC CAT TTT AAA AGT GGT GGA CCT ATA ACT TAT AAA GTT ATG AGA GAG 780

215 220 225
Val Lys Ala Leu Ala Tyr Phe Val Asn Gly Thr Ala His Asp Val Ile Leu Cys Asp Asp
GTT AAA GCC TTG GCT TAT TTT GTC AAT GGT ACT GCA CAT GAT GTC ATT CTA TGT GAT GAC 840

235 240 245
Thr Pro Arg Gly Leu Leu Ala Cys Gln Tyr Asn Thr Gly Asn Phe Ser Asp Gly Phe Tyr
ACA CCT AGA GGT TTG TTA GCA TGC CAA TAT AAT ACT GGC AAT TTT TCA GAT GGC TTC TAT 900

255 260 265
Pro Phe Thr Asn Thr Ser Ile Val Lys Asp Lys Phe Ile Val Tyr Arg Glu Ser Ser Val
CCT TTT ACT AAT ACT AGT ATT GTT AAG GAT AAG TTT ATT GTT TAT CGT GAA AGT AGT GTC 960

275 280 285
Asn Thr Thr Leu Thr Leu Thr Asn Phe Thr Phe Ser Asn Glu Ser Gly Ala Pro Pro Asn
AAT ACT ACT TTA ACA TTA ACT AAT TTC ACG TTT AGT AAT GAA AGT GGT GCC CCT CCT AAT 1020

295 300 305
Thr Gly Gly Val Asp Ser Phe Ile Leu Tyr Gln Thr Gln Thr Ala Gln Ser Gly Tyr Tyr
ACA GGT GGT GTT GAC AGT TTT ATT TTA TAC CAG ACA CAA ACA GCT CAG AGT GGT TAT TAT 1080

315 320 325
Asn Phe Asn Phe Ser Phe Leu Ser Ser Phe Val Tyr Arg Glu Ser Asn Tyr Met Tyr Gly
AAT TTT AAC TTT TCA TTT CTG AGT AGT TTT GTT TAT AGG GAA AGT AAT TAT ATG TAT GGA 1140

335 340 345
Ser Tyr His Pro Arg Cys Ser Phe Arg Pro Glu Thr Leu Asn Asn Gly Leu Trp Phe Asn
TCT TAC CAT CCA CGT TGT AGT TTT AGA CCT GAA ACC CTT AAT AAT GGT TTG TGG TTT AAT 1200

355 360 365
Ser Leu Ser Val Ser Leu Thr Tyr Gly Pro Ile Gln Gly Gly Cys Lys Gln Ser Val Phe
TCC CTT TCT GTT TCA TTA ACA TAC GGT CCC ATT CAA GGT GGT TGT AAG CAA TCT GTA TTT 1260

375 380 385
Asn Gly Lys Ala Thr Cys Cys Tyr Ala Tyr Ser Tyr Gly Gly Pro Arg Gly Cys Lys Gly
AAT GGT AAA GCA ACT TGT TGT TAT GCT TAT TCA TAC GGA GGA CCT CGT GGT TGT AAA GGT 1320

395 400 405
Val Tyr Arg Gly Glu Leu Thr Gln His Phe Glu Cys Gly Leu Leu Val Tyr Val Thr Lys
GTC TAT AGA GGT GAG CTA ACA CAG CAT TTT GAA TGT GGT TTG TTA GTT TAT GTT ACT AAG 1380

415 420 425
Ser Asp Gly Ser Arg Ile Gln Thr Ala Thr Gln Pro Pro Val Leu Thr Gln Asn Phe Tyr
AGC GAT GGC TCC CGT ATA CAA ACT GCA ACA CAA CCA CCT GTA TTA ACC CAA AAT TTT TAT 1440

435 440 445
Asn Asn Ile Asn Leu Gly Lys Cys Val Asp Tyr Asn Ile Tyr Gly Arg Ile Gly Gln Gly
AAT AAC ATC AAT TTA GGT AAG TGT GTT GAT TAT AAT ATA TAT GGC AGA ATT GGC CAA GGT 1500

455 460 465
Leu Ile Thr Asn Val Thr Asp Leu Ala Val Ser Tyr Asn Tyr Leu Ser Asp Ala Gly Leu
CTT ATT ACT AAT GTA ACC GAC TTA GCT GTT AGT TAT AAT TAT TTA TCA GAC GCA GGT TTG 1560

475 480 485
Ala Ile Leu Asp Thr Ser Gly Ala Ile Asp Ile Phe Val Val Gln Gly Glu Tyr Gly Pro
GCT ATT TTA GAT ACA TCT GGT GCC ATA GAC ATC TTC GTT GTA CAA GGT GAA TAT GGT CCT 1620

495 500 505
Asn Tyr Tyr Lys Val Asn Pro Cys Glu Asp Val Asn Gln Gln Phe Val Val Ser Gly Gly
AAC TAT TAT AAG GTT AAT CCA TGT GAA GAT GTC AAC CAA CAG TTT GTA GTT TCT GGT GGT 1680

515 520 525
Lys Leu Val Gly Ile Leu Thr Ser Arg Asn Glu Thr Gly Ser Gln Leu Leu Glu Asn Gln
AAA TTA GTA GGT ATT CTC ACT TCA CGT AAT GAA ACA GGT TCT CAG CTT CTT GAG AAC CAG 1740

535 540 545
Phe Tyr Ile Lys Ile Thr Asn Gly Thr Arg Arg Ser Arg Arg Ser Val Thr Glu Asn Val
TTT TAT ATT AAA ATC ACT AAT GGA ACT CGT CGT TCT AGA CGT TCT GTT ACT GAA AAT GTT 1800

555 560 565
Thr Asn Cys Pro Tyr Val Ser Tyr Gly Lys Phe Cys Ile Lys Pro Asp Gly Ser Ile Ser
ACA AAT TGC CCT TAT GTT AGT TAT GGC AAG TTT TGT ATA AAA CCT GAT GGT TCA ATT TCT 1860

575 580 585
Val Ile Val Pro Lys Glu Leu Asp Gln Phe Val Ala Pro Leu Leu Asn Val Thr Glu Tyr
GTA ATA GTA CCA AAA GAA CTG GAT CAG TTT GTG GCA CCT TTA CTT AAT GTT ACT GAA TAT 1920

595 600 605
Val Leu Ile Pro Asn Ser Phe Asn Leu Thr Val Thr Asp Glu Tyr Ile Gln Thr Arg Met
GTG CTC ATA CCT AAC AGT TTT AAT TTA ACT GTT ACA GAT GAG TAC ATA CAA ACG CGT ATG 1980

615 620 625
Asp Lys Ile Gln Ile Asn Cys Leu Gln Tyr Val Cys Gly Asn Ser Leu Ala Cys Arg Lys
GAT AAG ATC CAA ATT AAT TGC CTG CAG TAT GTT TGT GGC AAT TCT TTG GCC TGT AGA AAG 2040

635 640 645
Leu Phe Gln Gln Tyr Gly Pro Val Cys Asp Asn Ile Leu Ser Val Val Asn Ser Val Gly
CTG TTT CAA CAA TAT GGG CCT GTT TGT GAC AAC ATA TTG TCT GTA GTA AAT AGT GTT GGT 2100

655 660 665
Gln Lys Glu Asp Met Glu Leu Leu Asn Phe Tyr Ser Ser Thr Lys Pro Ala Arg Phe Asn
CAA AAA GAA GAT ATG GAA CTT TTA AAT TTC TAT TCT TCT ACT AAA CCA GCT CGT TTT AAT 2160

675 680 685
Thr Pro Val Phe Ser Asn Leu Ser Thr Gly Glu Phe Asn Ile Ser Leu Leu Leu Thr Pro
ACA CCA GTT TTT AGT AAT CTT AGC ACT GGT GAG TTT AAT ATT TCT CTT TTG TTA ACA CCC 2220

695 700 705
Pro Ser Ser Pro Arg Arg Arg Ser Phe Ile Glu Asp Leu Leu Phe Thr Ser Val Glu Ser
CCT AGT AGT CCT AGG AGG CGT TCT TTT ATT GAA GAT CTT TTA TTT ACA AGT GTT GAA TCT 2280

715 720 725
Val Gly Leu Pro Thr Asp Asp Ala Tyr Lys Lys Cys Thr Ala Gly Pro Leu Gly Phe Leu
GTA GGA TTA CCA ACA GAT GAC GCA TAC AAA AAG TGC ACT GCA GGA CCT TTA GGC TTT CTT 2340

735 740 745
Lys Asp Leu Ala Cys Ala Arg Glu Tyr Asn Gly Leu Leu Val Leu Pro Pro Ile Ile Thr
AAA GAC CTT GCA TGT GCT CGT GAA TAT AAT GGT TTG CTT GTG TTG CCT CCT ATT ATA ACA 2400

755 760 765
Ala Glu Met Gln Thr Leu Tyr Thr Ser Ser Leu Val Ala Ser Met Ala Phe Gly Gly Ile
GCA GAA ATG CAA ACT TTG TAT ACT AGT TCT TTA GTA GCT TCT ATG GCT TTT GGT GGT ATT 2460

775 780 785
Thr Ala Ala Gly Ala Ile Pro Phe Ala Thr Gln Leu Gln Ala Arg Ile Asn His Leu Gly
ACT GCA GCT GGT GCC ATA CCT TTT GCC ACA CAA CTG CAG GCT AGA ATT AAT CAC TTG GGT 2520

795 800 805
Ile Thr Gln Ser Leu Leu Leu Lys Asn Gln Glu Lys Ile Ala Ala Ser Phe Asn Lys Ala
ATT ACC CAG TCA CTT TTG TTG AAG AAT CAA GAA AAA ATT GCT GCT TCC TTT AAT AAG GCC 2580

815 820 825
Ile Gly His Met Gln Glu Gly Phe Arg Ser Thr Ser Leu Ala Leu Gln Gln Ile Gln Asp
ATT GGT CAT ATG CAG GAA GGT TTT AGG AGT ACA TCT CTA GCA TTA CAA CAA ATT CAA GAT 2640

835 840 845
Val Val Asn Lys Gln Ser Ala Ile Leu Thr Glu Thr Met Ala Ala Leu Asn Lys Asn Phe
GTT GTT AAT AAG CAG AGT GCT ATT CTT ACT GAG ACT ATG GCA GCA CTT AAT AAA AAT TTT 2700

855 860 865
Gly Ala Ile Ser Ser Val Ile Gln Asp Ile Tyr Gln Gln Leu Asp Ser Ile Gln Ala Asp
GGT GCT ATT TCT TCT GTG ATT CAA GAC ATT TAC CAG CAA CTT GAT TCC ATA CAA GCA GAT 2760

875 880 885
Ala Gln Val Asp Arg Leu Ile Thr Gly Arg Leu Ser Ser Leu Ser Val Leu Ala Ser Ala
GCT CAA GTG GAT CGG CTC ATA ACT GGT AGA TTG TCA TCA CTT TCT GTC TTA GCA TCT GCT 2820

895 900 905
Lys Gln Ser Glu Tyr Ile Arg Val Ser Gln Gln Arg Glu Leu Ala Thr Gln Lys Ile Asn
AAG CAG TCG GAG TAC ATT AGA GTG TCA CAA CAG CGT GAG TTA GCT ACT CAG AAA ATT AAT 2880

915 920 925
Glu Cys Val Lys Ser Gln Ser Ile Arg Tyr Ser Phe Cys Gly Asn Gly Arg His Val Leu
GAG TGT GTT AAA TCA CAG TCT ATT AGG TAT TCC TTT TGT GGT AAT GGA CGA CAT GTT TTA 2940

935 940 945
Thr Ile Pro Gln Asn Ala Pro Asn Gly Ile Val Phe Ile His Phe Thr Tyr Thr Pro Glu
ACC ATA CCA CAA AAT GCC CCT AAT GGT ATA GTG TTT ATA CAC TTT ACT TAT ACA CCA GAG 3000

955 960 965
Ser Phe Ile Asn Val Thr Ala Ile Val Gly Phe Cys Val Ser Pro Ala Asn Ala Ser Gln
AGC TTT ATT AAT GTT ACT GCA ATA GTG GGT TTT TGT GTA AGT CCT GCT AAT GCT AGT CAG 3060

975 980 985
Tyr Ala Ile Val Pro Ala Asn Gly Arg Gly Ile Phe Ile Gln Val Asn Gly Ser Tyr Tyr
TAT GCA ATA GTG CCC GCT AAT GGT AGG GGT ATT TTT ATA CAA GTT AAT GGT AGT TAC TAC 3120

995 1000 1005
Ile Thr Ala Arg Asp Met Tyr Met Pro Arg Asp Ile Thr Ala Gly Asp Ile Val Thr Leu
ATC ACT GCA CGA GAT ATG TAT ATG CCA AGA GAT ATT ACT GCA GGA GAT ATA GTT ACG CTT 3180

1015 1020 1025
Thr Ser Cys Gln Ala Asn Tyr Val Ser Val Asn Lys Thr Val Ile Thr Thr Phe Val Asp
ACT TCT TGT CAA GCA AAT TAT GTA AGT GTA AAT AAG ACC GTC ATT ACT ACA TTT GTA GAC 3240

1035 1040 1045
Asn Asp Asp Phe Asp Phe Asp Asp Glu Leu Ser Lys Trp Trp Asn Asp Thr Lys His Glu
AAT GAT GAT TTT GAT TTT GAT GAT GAA TTG TCA AAA TGG TGG AAT GAT ACT AAG CAT GAG 3300

1055 1060 1065
Leu Pro Asp Phe Asp Lys Phe Asn Tyr Thr Val Pro Ile Leu Asp Ile Asp Ser Glu Ile
CTA CCA GAC TTT GAC AAA TTC AAT TAC ACA GTA CCT ATA CTT GAC ATT GAT AGT GAA ATT 3360

1075 1080 1085
Asp Arg Ile Gln Gly Val Ile Gln Gly Leu Asn Asp Ser Leu Ile Asp Leu Glu Thr Leu
GAT CGT ATT CAA GGC GTT ATA CAG GGT CTT AAC GAC TCT CTA ATA GAC CTT GAA ACA CTA 3420

1095 1100 1105
Ser Ile Leu Lys Thr Tyr Ile Lys Trp Pro Trp Tyr Val Trp Leu Ala Ile Ala Phe Ala
TCA ATA CTC AAA ACT TAT ATT AAG TGG CCT TGG TAT GTG TGG TTA GCC ATA GCT TTT GCC 3480

1115                    1120                    1125
Thr Ile Ile Phe Ile Leu Ile Leu Gly Trp Cys Phe Met Thr Gly Cys Cys Gly Cys Cys
ACT ATT ATC TTC ATC TTA ATA CTA GGA TGG TGT TTC ATG ACT GGT TGT TGT GGT TGT TGT  3540

1135                    1140                    1145
Cys Gly Cys Phe Gly Ile Ile Pro Leu Met Ser Lys Cys Gly Lys Lys Ser Ser Tyr Tyr
TGT GGA TGC TTT GGC ATT ATT CCT TTA ATG AGT AAG TGT GGT AAG AAA TCT TCT TAT TAC  3600

1155                    1160                    1165
Thr Thr Phe Asp Asn Asp Val Val Thr Glu Gln Tyr Arg Pro Lys Lys Ser Val
ACG ACT TTT GAT AAT GAT GTG GTA ACT GAA CAA TAC AGA CCT AAA AAG TCT GTT  3654

TAATGATTCA AACTCCCACA TCTTTTCTAA TAGTATTAAT TTTTCTTTGG TGTAAACTTG  3714

CACTAAGTTG TTTTAAAGAG TGTGTTATAG CACTCCAGCA ACTAATACAA GTTTTACTCC  3774

AAATTATTAA TAGTAACTTA CAGTCTAGAG TCGACCTGCA GGCATGCAAG  3824

## Claims (Claims for the following Contracting State(s): BE, CH, LI, DE, FR, GB, IT, NL, SE)

1. A nucleic acid sequence encoding a polypeptide comprising the S1 subunit of the spike protein polypeptide of an Infectious Bronchitis Virus strain belonging to the Arkansas serotype having the amino acid sequence 1-539 shown in SEQ ID NO. 1.

2. A nucleic acid sequence according to claim 1 comprising the nucleotide sequence between about 151-1767 shown in SEQ ID NO. 1.

3. A nucleic acid sequence according to claim 1, encoding a polypeptide having the amino acid sequence 1-1168 shown in SEQ ID NO. 1.

4. A recombinant nucleic acid molecule comprising a nucleic acid sequence according to claims 1-3.

5. A recombinant vector virus containing a nucleic acid sequence according to claims 1-3.

6. A host cell transformed with the recombinant nucleic acid molecule according to claim 4 or infected with the recombinant vector virus according to claim 5.

7. A polypeptide having the amino acid sequence 1-539 or 1-1168 shown in SEQ ID NO. 1.

8. A vaccine for the protection of poultry against Infectious Bronchitis Virus infection comprising an immunogenically effective amount of an active ingredient selected from the group consisting of a recombinant vector virus according to claim 5, a host cell according to claim 6 and a polypeptide according to claim 7.

9. Method for the detection of the presence of IBV nucleic acid sequences derived from an IBV strain of the Arkansas serotype, characterized in that a nucleic acid sequence according to claims 1-3 are used in a hybridization test.

## Claims (Claims for the following Contracting State(s): ES, GR)

1. Method for the preparation of a nucleic acid sequence encoding a polypeptide comprising the S1 subunit of the spike protein polypeptide of an Infectious Bronchitis Virus strain belonging to the Arkansas serotype having the amino acid sequence 1-539 shown in SEQ ID NO. 1, which method comprises cloning of cDNA of said Infectious Bronchitis Virus strain and isolating said nucleic acid sequence from an appropriate clone.

2. Method according to claim 1, characterized in that the nucleic acid sequence comprises the nucleotide sequence between about 151-1767 shown in SEQ ID NO. 1.

3. Method according to claim 1, characterized in that the nucleic acid sequence encodes a polypeptide having the amino acid sequence 1-1168 shown in SEQ ID NO. 1.

4. Method for the preparation of a recombinant nucleic acid molecule, characterized in that a nucleic acid sequence prepared according to claims 1-3 is linked to another DNA.

5. Method for the preparation of a recombinant vector virus, characterized in that a nucleic acid sequence prepared according to claims 1-3 is inserted into a vector virus.

6. Method for the preparation of a host cell capable of expressing an Infectious Bronchitis Virus protein, said method comprising transformation of a suitable host cell with the recombinant nucleic acid molecule prepared according to claim 4

7. Method for the preparation of a host cell capable of expressing an Infectious Bronchitis Virus protein, said method comprising infection of a suitable host cell with the recombinant vector virus prepared according to claim 5.

8. Method for the preparation of a polypeptide, which method comprises
a) culturing a transformed host cell or recombinant vector virus comprising a nucleotide sequence encoding the amino acid sequence 1-539 or 1-1168 shown in SEQ ID NO. 1. under conditions in which the polypeptide is expressed and
b) isolating the polypeptide from the culture.

9. Method for the preparation of a vaccine for he protection of poultry against Infectious Bronchitis Virus infection, characterised in that an immunogenically effective amount of an active ingredient selected from the group of a recombinant vector virus prepared according to claim 5, a host cell prepared according to claim 6 or 7 and a polypeptide prepared according to claim 8 is mixed with a suitable carrier.

10. Method for the detection of the presence of IBV nucleic acid sequences derived from an IBV strain of the Arkansas serotype, characterised in that a nucleic acid sequence prepared according to claims 1-3 is used in a hybridization test.

## Patentansprüche (Patentansprüche für folgende(n) Vertragsstaat(en): BE, CH, LI, DE, FR, GB, IT, NL, SE)

1. Nukleinsäuresequenz, die für ein Polypeptid codiert, das die S1-Untereinheit des Spikeprotein-Polypeptids eines zum Arkansas-Serotyp gehörenden Virus der Infektiösen Bronchitis umfasst, welches die in der SEQ ID NO. 1 gezeigte Aminosäuresequenz 1-539 besitzt.

2. Nukleinsäuresequenz gemäss Anspruch 1, welche die in SEQ ID NO. 1 gezeigte Nukleotidsequenz zwischen ungefähr 151-1767 umfasst.

3. Nukleinsäuresequenz gemäss Anspruch 1, die für ein Polypeptid mit der in SEQ ID NO. 1 gezeigten Aminosäuresequenz 1-1168 codiert.

4. Rekombinantes Nukleinsäuremolekül, das eine Nukleinsäuresequenz gemäss Ansprüchen 1-3 umfasst.

5. Rekombinantes Vektorvirus, das eine Nukleinsäuresequenz gemäss Ansprüchen 1-3 enthält.

6. Wirtszelle, die mit dem rekombinanten Nukleinsäuremolekül gemäss Anspruch 4 transformiert oder mit dem rekombinanten Vektorvirus gemäss Anspruch 5 infiziert ist.

7. Polypeptid mit der in SEQ ID NO. 1 gezeigten Aminosäuresequenz 1-539 oder 1-1168.

8. Impfstoff zum Schutz von Geflügel vor Infektionen mit einem Virus der Infektiösen Bronchitis, der eine immunogen wirksame Menge eines aktiven Inhaltsstoffs enthält, der aus der Gruppe bestehend aus einem rekombinanten Vektorvirus gemäss Anspruch 5, einer Wirtszelle gemäss Anspruch 6 und einem Polypeptid gemäss Anspruch 7 ausgewählt wird.

9. Verfahren zum Nachweis der Anwesenheit von IBV-Nukleinsäuresequenzen, die von einem IBV-Stamm des Arkansas-Serotyps stammen, dadurch gekennzeichnet, dass eine Nukleinsäuresequenz gemäss Ansprüchen 1-3 in einem Hybridisierungstest verwendet wird.

## Patentansprüche (Patentansprüche für folgende(n) Vertragsstaat(en): ES, GR)

1. Verfahren zur Herstellung einer Nukleinsäuresequenz, die für ein Polypeptid codiert, welches die S1-Untereinheit des Spikeprotein-Polypeptids eines zum Arkansas-Serotyp gehörenden Virus des Infektiösen Bronchitis mit der in SEQ ID NO. 1 gezeigten Aminosäuresequenz 1-539 umfasst, wobei dieses Verfahren das Klonieren von cDNA des besagten Virusstammes der Infektiösen Bronchitis und die Isolierung besagter Nukleinsäuresequenz aus einem geeigneten Klon umfasst.

2. Verfahren gemäss Anspruch 1, dadurch gekennzeichnet, dass die Nukleinsäuresequenz die in SEQ ID NO. 1 gezeigte Nukleotidsequenz zwischen ungefähr 151-1767 umfasst.

3. Verfahren gemäss Anspruch 1, dadurch gekennzeichnet, dass die Nukleinsäuresequenz für ein Polypeptid mit der in SEQ ID NO. 1 gezeigten Aminosäuresequenz 1-1168 codiert.

4. Verfahren zur Herstellung eines rekombinanten Nukleinsäuremoleküls, dadurch gekennzeichnet, dass eine gemäss Ansprüchen 1-3 hergestellte Nukleinsäuresequenz mit anderer DNA verbunden wird.

5. Verfahren zur Herstellung eines rekombinanten Vektorvirus, dadurch gekennzeichnet, dass eine gemäss Ansprüchen 1-3 hergestellte Nukleinsäuresequenz in einen Vektorvirus eingefügt wird.

6. Verfahren zur Herstellung einer zur Expression eines Proteins des Virus der Infektiösen Bronchitis fähigen Wirtszelle, wobei besagtes Verfahren die Transformation einer geeigneten Wirtszelle mit dem gemäss Anspruch 4 hergestellten rekombinanten Nukleinsäuremolekül umfasst.

7. Verfahren zur Herstellung einer zur Expression eines Proteins des Virus der Infektiösen Bronchitis fähigen Wirtszelle, wobei besagtes Verfahren die Infektion einer geeigneten Wirtszelle mit dem gemäss Anspruch 5 hergestellten rekombinanten Vektorvirus umfasst.

8. Verfahren zur Herstellung eines Polypeptids, wobei besagtes Verfahren
a) Züchten einer transformierten Wirtszelle oder eines rekombinanten Vektorvirus, welche eine für die in SEQ ID NO. 1 gezeigte Aminosäuresequenz 1-539 oder 1-1168 codierende Nukleotidsequenz umfassen, unter Bedingungen, unter denen das Polypeptid exprimiert wird, und
b) Isolieren des Polypeptids aus der Kultur umfasst.

9. Verfahren zur Herstellung eines Impfstoffes zum Schutz von Geflügel vor Infektion mit dem Virus der Infektiösen Bronchitis, dadurch gekennzeichnet, dass eine immunogen wirksame Menge eines aktiven Inhaltsstoffes, welcher aus der Gruppe bestehend aus einem rekombinanten, gemäss Anspruch 5 hergestellten Virusvektor, einer gemäss Anspruch 6 oder 7 hergestellten Wirtszelle und einem gemäss Anspruch 8 hergestellten Polypeptid bestehenden Gruppe ausgewählt und mit einem geeigneten Träger gemischt wird.

10. Verfahren zum Nachweis der Anwesenheit von IBV-Nukleinsäuresequenzen, die von einem IBV-Stamm des Arkansas-Serotyps stammen, dadurch gekennzeichnet, dass eine gemäss Ansprüchen 1-3 hergestellte Nukleinsäuresequenz für einen Hybridisierungstest verwendet wird.

## Revendications (Revendications pour l'(les) Etat(s) contractant(s) suivant(s): BE, CH, LI, DE, FR, GB, IT, NL, SE)

1. Une séquence d'acide nucléique codant pour un polypeptide comprenant la sous-unité S1 du polypeptide d'une protéine de spicule d'une souche du virus de la bronchite infectieuse appartenant au sérotype Arkansas présentant la séquence des acides aminés 1 à 539 représentée dans SEQ ID N°1.

2. Une séquence d'acide nucléique selon la revendication 1, comprenant la séquence nucléotidique située environ entre les nucléotides 151 à 1767 représentée dans SEQ ID N°1.

3. Une séquence d'acide nucléique selon la revendication 1, codant pour un polypeptide présentant la séquence des acides aminés 1 à 1168 représentée dans SEQ ID N°1.

4. Une molécule d'acide nucléique recombinant comprenant une séquence nucléotidique selon les revendications 1 à 3.

5. Un virus vecteur recombinant contenant une séquence nucléotidique selon les revendications 1 à 3.

6. Une cellule hôte transformée par la molécule d'acide nucléique recombinant selon la revendication 4 ou infectée par le virus vecteur recombinant selon la revendication 5.

7. Un polypeptide présentant la séquence des acides aminés 1 à 539 ou 1 à 1168 représentée dans SEQ ID N°1.

8. Un vaccin pour la protection des volailles contre l'infection par le virus de la bronchite infectieuse comprenant une quantité efficace du point de vue immunologique d'un ingrédient actif sélectionné dans le groupe consistant en un virus vecteur recombinant selon la revendication 5, une cellule hôte selon la revendication 6 et un polypeptide selon la revendication 7.

9. Procédé de détection de la présence de séquences d'acide nucléique dérivées d'une souche d'IBV du sérotype Arkansas, caractérisé en ce qu'une séquence d'acide nucléique selon les revendications 1 à 3 est utilisée dans un test d'hybridation.

## Revendications (Revendications pour l'(les) Etat(s) contractant(s) suivant(s): ES, GR)

1. Procédé de préparation d'une séquence d'acide nucléique codant pour un polypeptide comprenant la sousunité S1 du polypeptide d'une protéine de spicule d'une souche du virus de la bronchite infectieuse appartenant au sérotype Arkansas présentant la séquence des acides aminés 1 à 539 représentée dans SEQ ID N°1, qui comprend le clonage d'ADNc de ladite souche du virus de la bronchite infectieuse et l'isolement de ladite séquence d'acide nucléique à partir d'un clone approprié.

2. Procédé selon la revendication 1, caractérisé en ce que la séquence d'acide nucléique comprend la séquence nucléotidique située environ entre le nucléotide 151 et le nucléotide 1767 représentée dans SEQ ID N°1.

3. Procédé selon la revendication 1, caractérisé en ce que la séquence d'acide nucléique code pour un polypeptide présentant la séquence des acides aminés 1 à 1168 représentée dans SEQ ID N°1.

4. Procédé de préparation d'une molécule d'acide nucléique recombinant, caractérisé en ce qu'une séquence d'acide nucléique préparée selon les revendications 1 à 3 est liée à un autre ADN.

5. Procédé de préparation d'un virus vecteur recombinant, caractérisé en ce qu'une séquence d'acide nucléique préparée selon les revendications 1 à 3 est insérée dans un virus vecteur.

6. Procédé de préparation d'une cellule hôte capable d'exprimer une protéine du virus de la bronchite infectieuse, ledit procédé comprenant la transformation d'une cellule hôte appropriée avec la molécule d'acide nucléique recombinant préparée selon la revendication 4.

7. Procédé de préparation d'une cellule hôte capable d'exprimer une protéine du virus de la bronchite infectieuse, ledit procédé comprenant l'infection d'une cellule hôte appropriée avec un virus vecteur recombinant préparé selon la revendication 5.

8. Procédé de préparation d'un polypeptide qui comprend:
(a) la culture d'une cellule hôte transformée ou d'un virus vecteur recombinant comprenant une séquence nucléotidique codant pour la séquence des acides aminés 1 à 539 ou 1 à 1168, représentée dans SEQ ID N°1 dans des conditions dans lesquelles le polypeptide est exprimé; et
(b) l'isolement du polypeptide à partir de la culture.

9. Procédé de préparation d'un vaccin pour la protection des volailles contre une infection par le virus de la bronchite infectieuse, caractérisé en ce qu'une quantité efficace du point de vue immunologique d'un ingrédient actif sélectionné dans le groupe comprenant un virus vecteur recombinant préparé selon la revendication 5, une cellule hôte préparée selon la revendication 6 ou 7 et un polypeptide préparé selon la revendication 8 est mélangé avec un support approprié.

10. Procédé de détection de la présence de séquences d'acide nucléique IBV dérivées d'une souche IBV du sérotype Arkansas, caractérisé en ce qu'une séquence d'acide nucléique préparée selon les revendications 1 à 3 est utilisée dans un test d'hybridation.
